# EUROPEAN PATENT SPECIFICATION

(11) **EP 2 819 583 B1**
(45) Date of publication and mention of the grant of the patent: **23.08.2017**
(21) Application number: 13714318.6
(22) Date of filing: 27.02.2013
(51) Int. Cl.: A61B 10/00, A61B 10/02

(54) **Biopsy device comprising a cell block material**
Biopsievorrichtung mit Zellenblockmaterial
Dispositif de biopsie avec un materiau de bloc de cellules

(30) Priority: 27.02.2012 GB 201203348
(43) Date of publication of application: 07.01.2015
(73) Proprietor: Exmoor Innovations Ltd, Taunton, Somerset TA1 2LB (GB)
(72) Inventor: EAST, Matthew, Taunton Somerset TA3 7AZ (GB); MAYALL, Frederick, Exeter Devon EX4 9JL (GB)
(74) Representative: Glansdorp, Freija Gwendolyn
(86) International application number: PCT/GB2013/050475
(87) International publication number: WO 2013/128177

(56) References cited:
- WO-A1-92/00039
- US-A- 3 224 434
- US-A1- 2007 166 834

## Description

### Field of the invention

The present invention relates to a device.

### Background to the invention

During medical diagnoses, it is often necessary to analyse cell samples taken from a patient, for example to diagnose tumours. Such diagnostic methods are commonly executed using cytological examination or histological examination. Cytological examination involves smearing or otherwise depositing a liquid sample of cells on to a glass slide and then examining them under a microscope. Histological examination involves taking a sample of solid tissue, fixing it, processing it, cutting a very thin section on to a glass slide and then examining it under a microscope.

One method of taking a sample of cells from a patient for cytological examination is fine needle aspiration (FNA). FNA is a diagnostic procedure commonly used to investigate lumps or masses, such as in the diagnosis of breast cancer and thyroid cancer. In this technique, single cells and small cell complexes are aspirated from a lesion with the aid of a thin needle with a hollow core, such as a hypodermic needle. The needle is introduced into the lesion and agitated to break up the cells in the lesion. There is a pressure difference between the subcutaneous tissues and the atmosphere outside, high to low respectively, that can be made greater by syringe suction. Due to a lower level of adhesion between cancer cells than between healthy cells, the cancer cell concentration in the sample may be enriched. Subsequent to taking the cell sample, the cell material is examined by e.g. ejecting them onto a glass slide, where they are smeared, fixed, stained and examined cytologically with a microscope.

Certain diagnostic techniques for analysing cells are best performed on a cell block. A cell block is a liquid cytology sample made solid. Cell blocks are generally prepared from FNA specimens or from serous fluid specimens such as pleural fluid (from the pleural cavity around the lungs) or ascitic fluid (from the cavity around the abdominal organs).

Known methods for preparing cell blocks from liquid cell samples involve wrapping a pellet of concentrated cells in thin paper or adding various solidifying materials, such as agar and thrombin, to a liquid concentrate of cells. HistoGel (Thermo Scientific, Loughborough, UK) is a commercially available cell block solidifying agent in which the primary ingredient is hydroxyethyl agarose. These solidifying agents are added to the sample as liquids and then left to solidify. Such known methods are described in Koss' diagnostic cytology and its histopathologic bases, Volume 2, Leopold G. Koss, Lippincott Williams & Wilkins, 2006, pages 1590-1592; and in US 5,137,710.

After solidification the cell block can be fixed and paraffin processed. Paraffin processing is a technique that is widely used to prepare organic tissue samples for microscopic examination. Organic tissue samples usually contain water and the technique involves replacing the water with paraffin wax. The replacement of the water with paraffin is usually achieved by first replacing the water with a hydrophilic organic solvent, such as ethanol. This organic solvent is then replaced, through a variety of steps which commonly involve the use of xylene, with hot liquid paraffin wax (melting point range 47 °C to 64 °C). Once the material has cooled after paraffin processing, it is possible to section the material into very thin sections, usually about 3 microns thick, without the sections breaking up, as the paraffin wax helps support the tissue's structure. An overview of this technique is given in The science of laboratory diagnosis, David Burnett, John Crocker, John Wiley & Sons, 2005, pages 7-11.

Mayall F.G. et al., J. Clin. Pathol. 2011, 64, 818-819 describes a method of making cytology cell blocks from serous fluid specimens using gelatin foam, which involves centrifuging the serous fluid in a universal container, removing the supernatant by pipette to leave a deposit of cells at the base of container, and absorbing these cells onto a crouton of gelatin foam, followed by fixation with alcohol or formalin. Serous fluid samples are not usually obtained by FNA techniques, but by inserting a drainage tube into a pleural cavity or abdominal cavity.

Cell block sections allow the study of additional morphological detail of a sample, such as, for example, the architecture of abnormal tissue or the fine detail of the cytoplasm and nuclei of the cells. Furthermore, they allow for the assessment of ancillary diagnostic tests such as immunohistochemistry and *in situ* hybridisation. For example, cell block immunohistochemistry and other molecular studies are often required to fully characterise tumours initially diagnosed by FNA cytology. These molecular investigations are becoming more important as they determine the treatment of tumours. For example, the drug Trastuzumab (Herceptin^{®}) is effective in treating breast carcinoma, but only if the carcinoma cells show amplification of human epidermal growth factor receptor 2 (HER2). The carcinoma cells have to be tested for Her2 amplification using immunohistochemistry and *in situ* hybridisation before treatment is planned and this testing is better done using cell block material than cell smear material.

In those cases in which histopathological examination or molecular studies are required, it is therefore usually necessary to take two separate cell samples from a patient: an FNA sample for cytopathological examination and then either another FNA sample for preparation as a cell block, or a biopsy. The biopsy can be an open biopsy, often requiring a general anaesthetic and admission to hospital, or a core biopsy. A core biopsy can usually be performed without a general anaesthetic or admission to hospital, but an FNA has some significant advantages over a core biopsy. The diameter of core biopsy instruments used to obtain cell blocks is several times greater than for FNA needles; needles used in core biopsies have diameters of up to 3.2 mm, as opposed to the needles most commonly used in FNA sampling which have an outer diameter of from 0.4 mm to 0.8 mm. It is important to note that an increase in diameter of the sampling needle is correlated to an increased risk of complications such as needle tract seeding, local bleeding and infection. In addition a core biopsy is much more painful than an FNA and core biopsy requires a local anaesthetic. An FNA usually can be performed without any anaesthetic.

US 2007/166834 A1 describes a sectionable cassette for use in a process for harvesting and handling tissue samples for biopsy analysis.

WO 92/00039 A1 describes a method for collecting and concentrating cells in a fluid such as body fluid, liquid for irrigation or aspiration cytological material, wherein the fluid is brought to pass through a brush or a wad of cotton in the flow passage of the liquid for collecting existing cells on the filter body.

US 3224434 A describes a device for, and a method of, obtaining samples of cells from the bodies of animate beings, such as human beings, for clinical observation and testing to determine the existence of disease, particularly cancer.

It is an aim of the present invention to provide a system which makes it possible to collect a cell block sample at the same time (i.e., simultaneously) as collecting an FNA sample, during the FNA procedure. This can enhance the accuracy of a diagnosis when limited sample is available, since there is almost no waste of sample material. It also relieves the need for using large diameter core biopsy needles, which reduces the risk of complications such as needle tract seeding, local bleeding and infection, and limits the discomfort of the patient. It also reduces the time of the sampling procedure and consequently reduces stress to the patient, and improves efficiency of the diagnostic process.

It is also an aim of the present invention to provide an improved method of obtaining a cell block sample which can enhance the ease and speed with which cell block samples can be obtained.

### Summary of the invention

According to the present invention there is provided a device comprising a housing and a cell block material, wherein the housing has a first end which is adapted to engage with sampling means *via* a gas tight fit, and at least part of the cell block material is positioned within the housing such that, in use, the cell block material is in fluid connection with the sampling means, characterized in that the cell block material protrudes from the first end of the housing such that, in use, the cell block material extends into the sampling means.

The cell block material is an absorbent material for forming cell blocks. It is preferably a substance that contains cavities within a solid matrix. It can be in the form of an open-cell foam, a fibrous material or a powder. In the case of a powder, the cell block material is formed so as to resemble a solid, for example by providing a membrane material to surround and shape the powder.

The cell block material is able to absorb a non-solid material such as, for example, a liquid or semi-liquid body fluid and/or tissue sample into its cavities.

The cell block material is a type of material which can be used to produce high quality sections for microscopic examination and molecular investigations, typically by paraffin processing to form a block. In order for the cell block material to be successfully paraffin processed and in order to produce high quality sections, it is necessary that the paraffin is able to penetrate not only into the cavities in the cell block material but also into the solid matrix of the walls of the cavities in the cell block material. If this is not achieved, the sections tend to fracture and break apart. In an embodiment, the cell block material should, therefore, be able to absorb paraffin wax into its solid matrix.

For paraffin processing to cause paraffin wax to penetrate into the solid matrix of the cell block material, it is desirable that a liquid, such as, for example, water, is present in the solid matrix of the cell block material when processing starts. The processing then draws paraffin wax into the solid matrix of the cell block material. In an embodiment, the cell block material should, therefore, also be able to absorb water into its solid matrix. In addition, the cell block material should not collapse, dissolve or melt during paraffin processing. In these ways the cell block material ideally closely resembles the respective properties of organic tissue. The cell block material preferably, therefore, has a melting point above about 64 °C. The cell block material should also be resistant to the solvents used during paraffin processing; as explained above, these solvents can for example include water, a hydrophilic organic solvent (such as ethanol) and/or xylene.

The sampling means can be any means through which a sample of cells can be drawn into the housing of the device. Examples include a needle, a pipette tip and tubing.

The housing has a first end which is adapted to engage with the sampling means. Depending on the choice of sampling means, the sampling means may have a particular component which is suitable for engaging with the first end of the housing. For example, the first end may be adapted to engage with a needle hub, which needle hub may form part of a needle. A needle as may be used in a medical and/or surgical setting commonly comprises a needle hub and a needle shaft. In use, a sample of cells would commonly be drawn up through the needle shaft into the needle hub.

In the case where the first end of the housing is adapted to engage with a needle hub, it is therefore suitable for engagement with a needle. The first end may also be suitable for engagement with another device such as an endoscope or tubing which may be useful to extract a tissue or fluid sample.

The term "fluid connection", as in the fluid connection between the cell block material and the sampling means (e.g. between the cell block material and a needle hub) in use, indicates that the cell block material is positioned in such a way that, in use, fluid which enters the sampling means (e.g. which enters a needle hub from a needle shaft) comes into contact with the cell block material. When the device is used under a negative pressure generated by, for example, pulling a syringe attached to the device, any air which enters the device from the sampling means (e.g. a needle) will flow e.g. through the needle hub and into the cell block material. Similarly, any other non-solid material, such as, for example, a liquid or semi-liquid body fluid and/or tissue sample, which enters the device through the sampling means (e.g. a needle) under a negative pressure will flow e.g. through the needle hub and into the cell block material.

The cell block material protrudes from the first end of the housing such that, in use, the cell block material extends into the sampling means, e.g. into a needle hub. In the case of a needle, this allows the cell block material to extend from the device housing to be in close proximity to or contact the end of the needle shaft which sits inside the needle hub, which means any sample emerging from the needle shaft may be absorbed by the cell block material and hence there is almost no waste of sample material.

The device according to the invention allows cell block samples to be collected directly while performing FNAs. During normal FNA operation, often a significant amount of the sample is left behind in the hub of the needle when the specimen is ejected onto slides for cytology smear analysis. The device of the invention collects this material during the FNA procedure, by being able to capture the overflow from the needle at the needle hub. The device of the invention therefore allows cell block samples to be collected while performing FNAs with little extra effort and little if any reduction in the quality of the FNA cytology smear specimen. Immunohistochemistry and other molecular investigations can be performed on the obtained cell block as for routine histology specimens. An adequate cytology smear specimen and cell block specimen can be collected simultaneously in the same FNA attempt, during a single procedure.

If a sample of cells is obtained directly from a subject, the device of the invention can allow the sample to be collected into the cell block material in a way that enhances the accuracy of interpretation. For example, the standard method of collecting an FNA sample causes all of the material that is collected at various stages of the procedure to be blended together; commonly, a high quality diagnostic sample of cells might be obtained early in in the procedure, but as sampling progresses the sample becomes increasingly contaminated with haemorrhage. An example of this would be the aspiration of a colloid cyst of thyroid: early on in the procedure clear golden colloid fluid is obtained, but later the fluid becomes haemorrhagic. Using the standard method, the haemorrhage will be blended through the entire sample by the time it is examined, giving the misleading impression that the entire sample was haemorrhagic. However, when using the device according to the invention, the sample can be retained in the cell block material in the order in which it was obtained, with the early sample being most distant from the sampling means and the late sample being closest to the sampling means.

The cell block material in the device according to the invention can also act as a partial filter. Larger particles may come to rest in the cell block material closest to the sampling means, while smaller particles and fluid penetrate further into the cell block material. This splitting of cells by size within the cell block material can be useful for diagnostic purposes as it can, for example, provide information about a type of tumour.

In an embodiment, the first end of the housing is adapted to engage with a needle hub. This results in a device comprising a housing and a cell block material, wherein the housing has a first end which is adapted to engage with a needle hub *via* a gas tight fit, at least part of the cell block material is positioned within the housing such that, in use, the cell block material is in fluid connection with the needle hub, and the cell block material protrudes from the first end of the housing such that, in use, the cell block material extends into the needle hub.

In an embodiment, the cell block material is a polymeric material.

In an embodiment, the cell block material is an open-cell foam. The foam is a substance which contains interlinked cells or cavities (pores) within a solid matrix. In an embodiment, the cavities have a pore size of up to 1 mm, or up to 0.5 mm. In an embodiment, the cavities have a pore size of at least 0.1 mm. In an embodiment, the cavities have a pore size of from 0.1 mm to 1 mm, or from 0.1 mm to 0.5 mm.

In an embodiment, the cell block material is a synthetic material, such as, for example, a synthetic foam. Suitable synthetic foams can provide a number of advantages over biological foams, such as having very little risk of carrying prions, being easier to sterilise, avoiding religious and cultural issues related to pig and beef derived products, being easier to license as a medical device, not containing contaminant animal DNA that could confuse analysis of the sample, being cheaper, being more robust, being rigid when dry and easier to machine, having a higher deformation temperature and higher melting temperature, having faster wicking (i.e. more affinity for a liquid) and/or being easily available in a wide variety of pore sizes.

In an embodiment, the solid matrix of the cell block material is formed by polyvinyl alcohol (PVA), cellulose acetate, methyl cellulose or carboxymethyl cellulose, or any combination thereof. In an embodiment, the solid matrix of the cell block material is formed by polyvinyl alcohol (PVA). In an embodiment, the solid matrix of the cell block material is formed by cellulose acetate. In an embodiment, the solid matrix of the cell block material is formed by methyl cellulose. In an embodiment, the solid matrix of the cell block material is formed by carboxymethyl cellulose.

In an embodiment, the cell block material is selected from cellulose sponge and PVA foam.

In an embodiment, the cell block material is cellulose sponge. Cellulose sponge is made from cellulose acetate.

In an embodiment, the cell block material is PVA foam. In addition to the advantages of a synthetic foam outlined above, PVA foam has some particular properties that make it ideal for paraffin processing, such as the fact that it is resistant to organic solvents. Furthermore, despite it being a hard material in the unprocessed state, it was surprisingly found that it allowed paraffin wax to penetrate into the solid matrix of the PVA foam on paraffin processing, allowing it to subsequently be sectioned smoothly without fragmenting. Without being bound by theory, this is thought to be caused by its hydrophilic properties, allowing it to absorb water into its solid matrix. As mentioned above, the water can be replaced with paraffin by first replacing the water with a hydrophilic organic solvent, such as ethanol, and then replacing this organic solvent, through a variety of steps which commonly involve the use of xylene, with hot liquid paraffin wax. Overall, therefore, it is thought that the property of allowing water to penetrate its solid matrix also allows paraffin wax to penetrate into the solid matrix of the foam on paraffin processing.

In an embodiment, the cell block material is a natural material, such as, for example, natural sponge or gelatin. In an embodiment, the cell block material is natural sponge. In an embodiment, the solid matrix of the cell block material is formed by gelatin. In an embodiment, the cell block material is gelatin foam.

In an embodiment, the first end of the housing is adapted to engage with the sampling means (e.g. with a needle hub) *via* an interference fit.

In an embodiment, the first end of the housing is adapted to engage with the sampling means (e.g. with a needle hub) *via* a Luer taper. A Luer taper is a common standardized system of fluid fittings used for making airtight connections between a male part on one device and its mating female part on another device. There are two varieties of Luer taper connections: Luer-Slip^{®} and Luer-Lok^{®}. In an embodiment, the first end is adapted to engage with the sampling means (e.g. with a needle hub) *via* a Luer-Slip^{®} connection. In an embodiment, the first end is adapted to engage with the sampling means (e.g. with a needle hub) *via* a Luer-Lok^{®} connection.

In an embodiment, the housing has a second end which is adapted to engage with a syringe nozzle *via* a gas tight fit. This makes it possible to fit the device in between a needle and a syringe. The cell sample can therefore be drawn up into the cell block material by using negative pressure provided by pulling the syringe. Other suction or extraction techniques are well-known to the skilled person.

In an embodiment, the second end of the housing is adapted to engage with the syringe nozzle *via* an interference fit.

In an embodiment, the second end of the housing is adapted to engage with the syringe nozzle *via* a Luer taper. In an embodiment, the second end is adapted to engage with the needle hub *via* a Luer-Slip^{®} connection. In an embodiment, the second end is adapted to engage with the needle hub *via* a Luer-Lok^{®} connection.

In an embodiment, the housing is a tubular housing which circumferentially surrounds or substantially circumferentially surrounds the cell block material.

In an embodiment, the cell block material is slidable within the housing such that, in use, it is able to slide further into the housing when the sampling means (e.g. a needle hub) is connected to the device. In the case where the sampling means is a needle, as the needle hub is placed on the device, the initially protruding cell block material slides further into the housing under the pressure from the needle hub as well as the needle shaft which emerges in the needle hub. Since standard needle hubs come in different depths, this embodiment allows the device to be used easily with needle hubs of different depths, while still resulting in a close proximity or a contact between the cell block material and the needle shaft after the needle hub has been connected to the device.

In an embodiment, the cell block material is shaped substantially like a cylinder and, in use with a needle, the longitudinal axis of the cylinder extends in substantially the same direction as the longitudinal axis of a needle shaft attached to the needle hub. The distal end of the foam cylinder, which in use with a needle extends from the device housing's first end and protrudes into the needle hub may, for example, be tapered or conical. The tapered or conical end may, in use with a needle, be in contact with the needle shaft inside the needle hub. The tapered or conical end may improve the cell block material's ability to act like a wick, allowing it to draw a sample out of the needle shaft and into the cell block material.

Also described is a device according to the invention, for use in a method of simultaneously obtaining FNA and cell block samples.

Also described is a cell block sample comprising sample cells, paraffin wax and a synthetic open-cell foam.

In an embodiment, the synthetic open-cell foam is a polymeric material.

The open-cell foam is a substance which contains interlinked cells or cavities (pores) within a solid matrix. In an embodiment, the cavities have a pore size of up to 1 mm, or up to 0.5 mm. In an embodiment, the cavities have a pore size of at least 0.1 mm. In an embodiment, the cavities have a pore size of from 0.1 mm to 1 mm, or from 0.1 mm to 0.5 mm.

In an embodiment, the solid matrix of the synthetic open-cell foam is formed by polyvinyl alcohol (PVA), cellulose acetate, methyl cellulose or carboxymethyl cellulose, or any combination thereof. In an embodiment, the solid matrix of the synthetic open-cell foam is formed by polyvinyl alcohol (PVA). In an embodiment, the solid matrix of the synthetic open-cell foam is formed by cellulose acetate. In an embodiment, the solid matrix of the synthetic open-cell foam is formed by methyl cellulose. In an embodiment, the solid matrix of the synthetic open-cell foam is formed by carboxymethyl cellulose.

In an embodiment, the synthetic open-cell foam is selected from cellulose sponge and PVA foam. In an embodiment, the synthetic open-cell foam is cellulose sponge. Cellulose sponge is made from cellulose acetate. In an embodiment, the synthetic open-cell foam is PVA foam.

In an embodiment, the sample cells comprise candidate tumour cells or other cells from potentially diseased tissue.

In an embodiment, the sample cells are obtainable from a bodily fluid such as, for example, blood, saliva, urine, cerebrospinal fluid, amniotic fluid, lymph fluid, pleural fluid or ascitic fluid.

In an embodiment, the sample cells are obtainable from a subject.

Also described is a method of obtaining a cell block sample, comprising the step of (i) absorbing a non-solid sample of cells into a solid cell block material to obtain a cell block sample, wherein the cell block material is not gelatin foam.

In an embodiment, the cell block material is a polymeric material.

In an embodiment, the cell block material is an open-cell foam. The foam is a substance which contains interlinked cells or cavities (pores) within a solid matrix. In an embodiment, the cavities have a pore size of up to 1 mm, or up to 0.5 mm. In an embodiment, the cavities have a pore size of at least 0.1 mm. In an embodiment, the cavities have a pore size of from 0.1 mm to 1 mm, or from 0.1 mm to 0.5 mm.

In an embodiment, the cell block material is a synthetic material such as, for example, a synthetic foam. As mentioned above, suitable synthetic foams can provide a number of advantages over biological foams, such as having very little risk of carrying prions, being easier to sterilise, avoiding religious and cultural issues related to pig and beef derived products, being easier to license as a medical device, not containing contaminant animal DNA that could confuse analysis of the sample, being cheaper, being more robust, being rigid when dry and easier to machine, having a higher deformation temperature and higher melting temperature, having faster wicking (i.e. more affinity for a liquid) and/or being easily available in a wide variety of pore sizes.

In an embodiment, the solid matrix of the cell block material is formed by polyvinyl alcohol (PVA), cellulose acetate, methyl cellulose or carboxymethyl cellulose, or any combination thereof. In an embodiment, the solid matrix of the cell block material is formed by polyvinyl alcohol (PVA). In an embodiment, the solid matrix of the cell block material is formed by cellulose acetate. In an embodiment, the solid matrix of the cell block material is formed by methyl cellulose. In an embodiment, the solid matrix of the cell block material is formed by carboxymethyl cellulose.

In an embodiment, the cell block material is selected from cellulose sponge and PVA foam.

In an embodiment, the cell block material is cellulose sponge. Cellulose sponge is made from cellulose acetate.

In an embodiment, the cell block material is PVA foam. In addition to the advantages of a synthetic foam outlined above, PVA foam has some particular properties that make it ideal for paraffin processing, such as the fact that it is resistant to organic solvents. Furthermore, despite it being a hard material in the unprocessed state, it was surprisingly found that it allowed paraffin wax to penetrate into the solid matrix of the PVA foam on paraffin processing, allowing it to subsequently be sectioned smoothly without fragmenting. Without being bound by theory, this is thought to be caused by its hydrophilic properties, allowing it to absorb water into its solid matrix. As mentioned above, the water can be replaced with paraffin by first replacing the water with a hydrophilic organic solvent, such as ethanol, and then replacing this organic solvent, through a variety of steps which commonly involve the use of xylene, with hot liquid paraffin wax. Overall, therefore, it is thought that the property of allowing water to penetrate its solid matrix also allows paraffin wax to penetrate into the solid matrix of the foam on paraffin processing.

In an embodiment, the cell block material is a natural material. In an embodiment, the cell block material is natural sponge.

In step (i) of the method of obtaining a cell block sample described above, a non-solid sample of cells is absorbed into a solid cell block material to obtain a cell block sample. This may be achieved in different ways.

In an embodiment, the non-solid sample of cells is absorbed into the solid cell block material by uplifting the sample of cells from a surface. In an embodiment, the surface is a non-living surface, such as for example a microscope slide.

In an embodiment, the cell block material is present inside a syringe.

In an embodiment, the cell block material forms a component of the device according to the invention.

In an embodiment, the non-solid sample of cells is located in a container and the solid cell block material is added into the container.

In an embodiment, the sample of cells comprises candidate tumour cells or other cells from potentially diseased tissue.

In an embodiment, the sample of cells is obtainable from a bodily fluid such as, for example, blood, saliva, urine, cerebrospinal fluid, amniotic fluid, lymph fluid, pleural fluid or ascitic fluid.

In an embodiment, the sample of cells is obtainable from a subject. In an embodiment, the method of obtaining a cell block sample described above also includes the step of obtaining the sample of cells from a subject. In an alternative embodiment, the method of obtaining a cell block sample described above does not include the step of obtaining the sample of cells from a subject.

The sample of cells may, for example, be obtained from a subject via fine needle aspiration (FNA), such as FNA of superficial lumps or masses, or the sample of cells may, for example, be obtained by taking a blood sample or a saliva swab. In addition, the sample of cells may, for example, be obtained from urine by collection from a voided specimen or during cystoscopy (endoscopic examination of the urinary tract); from cerebrospinal fluid by needle puncture aspiration (lumbar puncture); from amniotic fluid by needle puncture aspiration; from pleural fluid by needle puncture aspiration of the chest cavity; or from ascitic fluid by needle puncture aspiration of the abdominal cavity.

In an embodiment, the method of obtaining a cell block sample described above further comprises the step of (ii) processing the cell block sample by fixing and paraffin processing.

Also described is a method of simultaneously obtaining FNA and cell block samples, comprising the step of: (i) attaching a device to a needle, wherein the device comprises a housing and a cell block material, the housing has a first end which is adapted to engage with a needle hub *via* a gas tight fit, and at least part of the cell block material is positioned within the housing such that, in use, the cell block material is in fluid connection with the needle hub.

In an embodiment, the cell block material protrudes from the first end of the housing such that, in use, the cell block material extends into the needle hub.

In an embodiment, the device is a device according to the invention.

In an embodiment, the method of simultaneously obtaining FNA and cell block samples described above further comprises the step of: (ii) collecting cells from a patient through the needle, during which step the overflow of cells from the needle is absorbed into the cell block material.

In an embodiment, the cells are candidate tumour cells or other cells from potentially diseased tissue.

Throughout the description and claims of this specification, the words "comprise" and "contain" and variations of the words, for example "comprising" and "comprises", mean "including but not limited to", and do not exclude other moieties, additives, components, integers or steps. Moreover the singular encompasses the plural unless the context otherwise requires: in particular, where the indefinite article is used, the specification is to be understood as contemplating plurality as well as singularity, unless the context requires otherwise.

Preferred features of each aspect of the invention may be as described in connection with any of the other aspects. Other features of the invention will become apparent from the following examples. Generally speaking, the invention extends to any novel one, or any novel combination, of the features disclosed in this specification (including any accompanying claims and drawings). Thus, features, integers, characteristics, compounds, chemical moieties or groups described in conjunction with a particular aspect, embodiment or example of the invention are to be understood to be applicable to any other aspect, embodiment or example described herein, unless incompatible therewith. Moreover, unless stated otherwise, any feature disclosed herein may be replaced by an alternative feature serving the same or a similar purpose.

Where upper and lower limits are quoted for a property, then a range of values defined by a combination of any of the upper limits with any of the lower limits may also be implied.

### Specific description

Embodiments of the present invention will now be further described with reference to the accompanying figures, of which:
Figure 1 shows a schematic cross-sectional side view of an embodiment of the device according to the invention.
Figure 2 shows a schematic cross-sectional side view of the device from Figure 1, in which the foam cylinder has been moved further into the housing.
Figure 3 shows a schematic cross-sectional side view of a needle, comprising a needle shaft and a needle hub.
Figure 4 shows a schematic cross-sectional side view of an assembly comprising the needle as shown in Figure 3, the embodiment of the device from Figure 1, and the distal part of a syringe including the syringe nozzle.
Figure 5 shows a perspective view of an assembly comprising a needle, an embodiment of the device of the invention and a syringe.
Figure 6 shows an enlarged perspective view of part of the assembly from Figure 5, comprising a needle, an embodiment of the device of the invention and the distal part of a syringe including the syringe nozzle.
Figure 7 shows a foam cylinder after it has been fixed and pulled from the housing.
Figure 8 shows a cell block section, prepared from the foam cylinder in Figure 7 after it has been paraffin processed and sectioned.
Figure 9 is a microscope image showing cells in the cavities of the foam matrix in the cell block section shown in Figure 8.
Figure 10 is a microscope image showing cells in the cavities of the foam matrix in the cell block section shown in Figure 8 after immunohistochemical staining for cytokeratin 7.

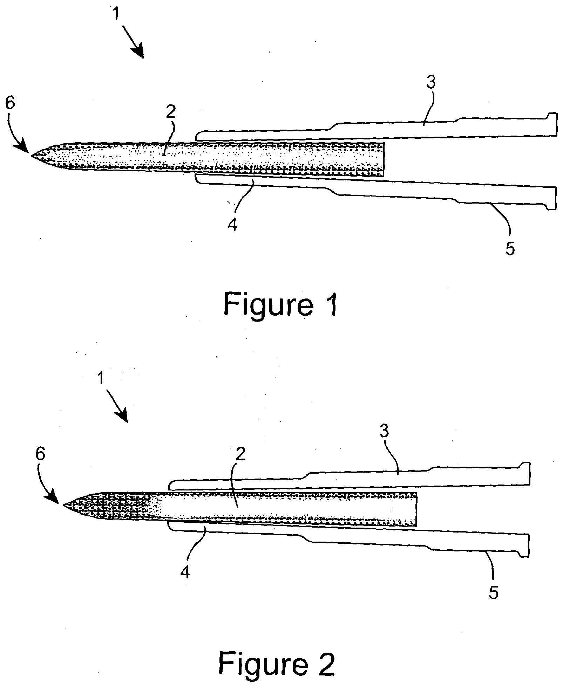

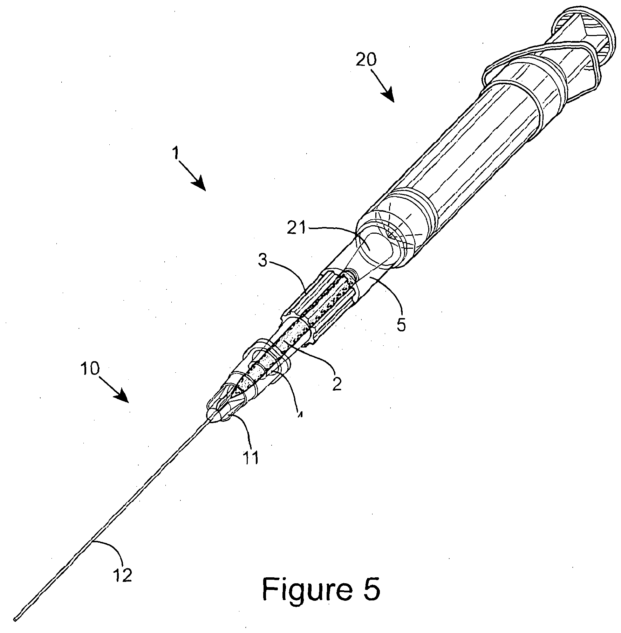

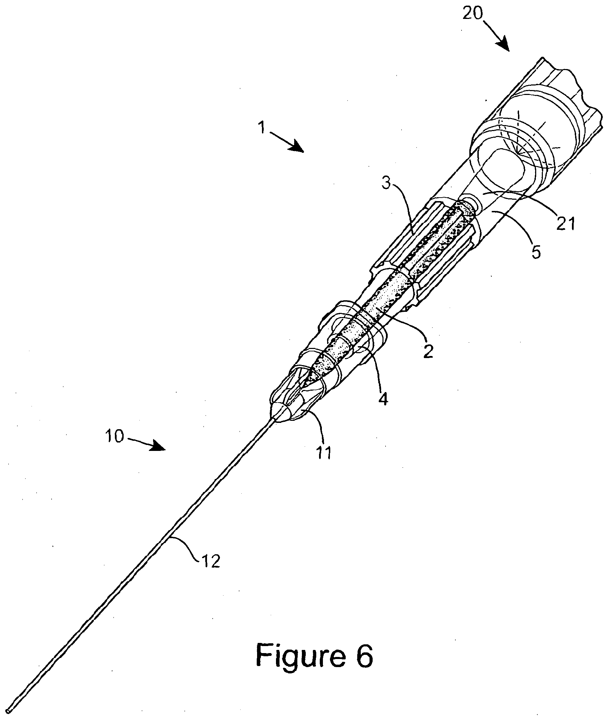

The device **1** shown in Figures 1, 2 and 4 to 6 comprises a cylinder of PVA foam **2,** which is held in a tubular housing **3.** In the embodiment shown, the distal end **6** of the cylinder has a conical shape.

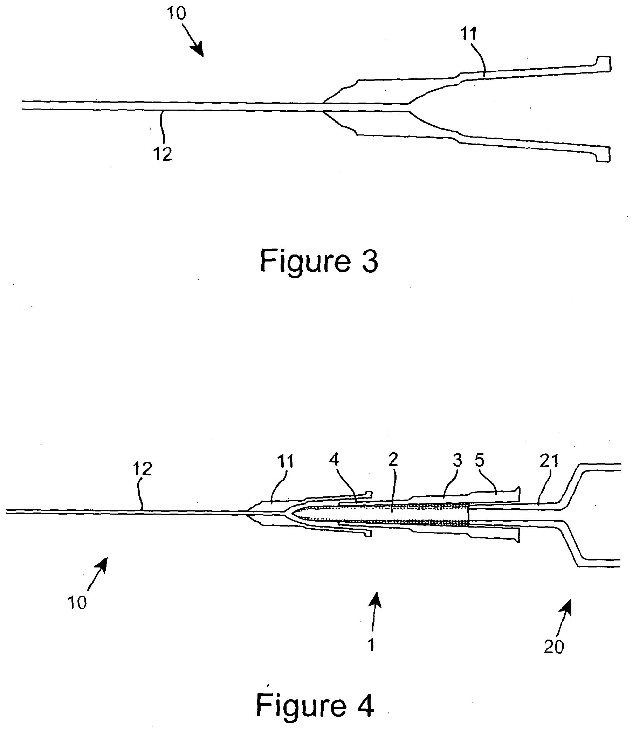

As shown in Figure 3, a standard needle **10** comprises a needle hub **11** and a needle shaft **12.**

As shown in Figures 1, 2 and 4 to 6, the device **1** has a housing **3** with a first end **4** which is adapted to engage with a needle hub **11** *via* a Luer taper. The housing **3** has a second end **5** which is adapted to engage with a syringe nozzle **21** *via* a Luer taper.

The PVA foam **2** protrudes from the first end **4** of the housing **3** in such a way that, when assembled with a needle **10,** it extends into the needle hub **11.** The foam **2** is slidable within the housing **3** so that, in use, it is able to slide further into the housing **3** when the needle hub **11** is connected to the device **1.** Figure 2 shows a situation where the foam **2** has been moved further into the device **1** than in Figure 1.

In use, as shown in Figures 4 to 6, the needle **10** is attached to the device **1** by connecting the needle hub **11** to the housing's first end **4.** The foam 2 protrudes from the housing **3** into the needle hub **11.** On attaching the needle **10,** the foam **2** can slide back into the housing **3,** resulting in the tip of the foam **2** extending into the apex of the needle hub **11.** The foam **2** is in fluid connection with the needle hub **11** so that any fluid entering the needle hub **11** can make contact with the foam **2.** The foam **2** may be in direct contact with the proximal end of the needle shaft **12** extending through the needle hub **11.** The axis of the foam cylinder **2** extends in substantially the same direction as that of the needle shaft **12** attached to the needle hub **11.**

If a syringe suction technique is used, the second end **5** of the housing **3** is connected to a syringe **20.** Figures 4 to 6 show an assembly comprising a needle **10,** the device **1** and a syringe **20.**

As the FNA is being performed, the sample material flows up the needle shaft **12** and, if a sufficient volume is collected, emerges into the needle hub **11** where it makes contact with and is absorbed into the tip of the PVA foam **2.** When FNA sampling is finished, smears are made by ejecting the specimen from the needle **10** onto a slide in the usual way by air pressure from the attached syringe **20.** During this ejection, the air passes through the foam **2** which may force some of the sample in the foam **2** back down the needle **10,** but some remains behind in the foam **2.** The device **1** is then detached from the needle **10** and the syringe **20.** The device **1** is then placed in a formalin specimen pot and sent for histopathology. Alternatively, the device **1** can be removed before the application of air pressure from the syringe **20,** so none of the sample in the foam **2** is discharged. In that case the total sample for the smears is derived from the liquid within the needle **10.**

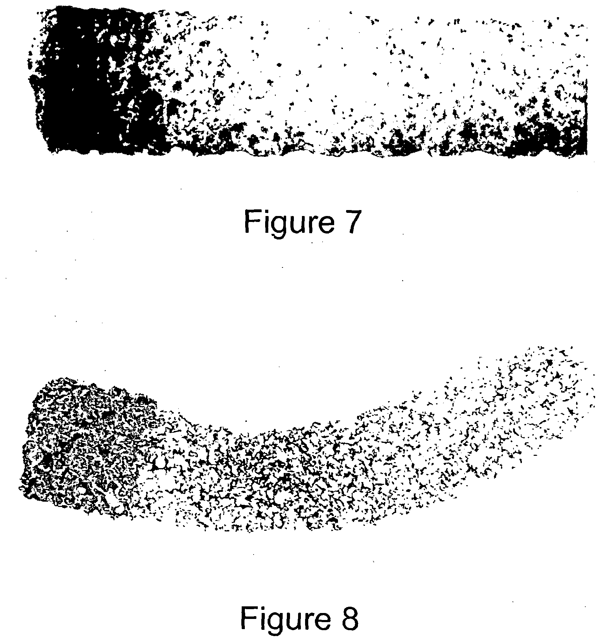

Before or after fixing, the PVA foam **2** is removed from the housing **3.** Figure 7 shows the foam cylinder **2** after it has been fixed and removed from the housing. The fixed PVA foam is then prepared by paraffin processing and sectioned as for routine histology specimens, resulting in a cell block section as shown in Figure 8.

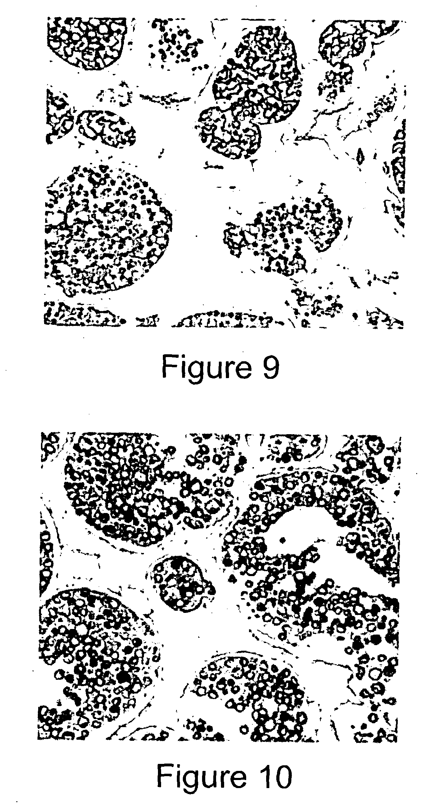

The sections can be stained with, for example, haemotoxylin and eosin and then, on microscopic examination, cells are seen in the cavities in the foam matrix, as shown in Figure 9. Immunohistochemistry and other molecular investigations can be performed as for routine histology specimens. Figure 10, for example, shows adenocarcinoma cells after immunohistochemical staining for cytokeratin 7; the cells are strongly positive. Cytokeratin 7 is typically positive in breast carcinomas, lung carcinomas, stomach carcinomas and pancreatic carcinomas.

An adequate cytology smear specimen and cell block specimen can be collected by the same FNA attempt. If preservation of high quality DNA is a priority, the part of the foam core bearing the sample may be cut in two with a scalpel, with one part being frozen for DNA studies while the rest is formalin fixed and paraffin processed.

## Claims

1. A device (1) comprising a housing (3) and a cell block material (2), wherein
the housing (3) has a first end (4) which is adapted to engage with sampling means (10) *via* a gas tight fit, and'
at least part of the cell block material (2) is positioned within the housing (3) such that, in use, the cell block material (2) is in fluid connection with the sampling means (10),
**characterized in that** the cell block material (2) protrudes from the first end (4) of the housing (3) such that, in use, the cell block material (2) extends into the sampling means (10).

2. The device of claim 1, wherein the first end (4) is adapted to engage with a needle hub (11).

3. The device of claim 1 or 2, wherein the cell block material (2) is a polymeric material.

4. The device of any one of claims 1 to 3, wherein the cell block material (2) is an open-cell foam.

5. The device of any one of claims 1 to 4, wherein the cell block material (2) is a synthetic material.

6. The device of claim 5, wherein the cell block material (2) is selected from cellulose sponge and PVA foam.

7. The device of claim 6, wherein the cell block material (2) is PVA foam.

8. The device of any one of the preceding claims, wherein the cell block material (2) is slidable within the housing (3) such that, in use, it is able to slide further into the housing (3) when the sampling means (10) is connected to the device (1).

9. The device of any one of the preceding claims, wherein the first end (4) of the housing (3) is adapted to engage with the sampling means (10) *via* an interference fit.

10. The device of any one of the preceding claims, wherein the first end (4) of the housing (3) is adapted to engage with the sampling means (10) *via* a Luer taper.

11. The device of any one of the preceding claims, wherein the housing (3) has a second end (5) which is adapted to engage with a syringe nozzle (21) *via* a gas tight fit.

12. The device of claim 11, wherein the second end (5) of the housing (3) is adapted to engage with the syringe nozzle (21) *via* an interference fit.

13. The device of claims 11 or 12, wherein the second end (5) of the housing (3) is adapted to engage with the syringe nozzle (21) *via* a Luer taper.

14. The device of any one of the preceding claims, wherein the housing (3) is a tubular housing which circumferentially surrounds or substantially circumferentially surrounds the cell block material (2).

15. The device of claim 2, wherein the cell block material (2) is shaped substantially like a cylinder and wherein, in use with a needle (10), the longitudinal axis of the cylinder extends in substantially the same direction as the longitudinal axis of a needle shaft (12) attached to the needle hub (11).

## Patentansprüche

1. Vorrichtung (1), umfassend ein Gehäuse (3) sowie ein Zellblockmaterial (2), wobei das Gehäuse (3) ein erstes Ende (4) aufweist, das ausgebildet ist, mit einem Probenentnahmemittel (10) über eine gasdichte Passung in Eingriff zu treten, und zumindest ein Teil des Zellblockmaterials (2) so innerhalb des Gehäuses (3) positioniert ist, dass sich bei Verwendung das Zellblockmaterial (2) mit den Probenentnahmemitteln (10) in Fluidverbindung befindet,
**dadurch gekennzeichnet, dass** das Zellblockmaterial (2) so von dem ersten Ende (4) des Gehäuses (3) vorsteht, dass sich bei Verwendung das Zellblockmaterial (2) in das Probenentnahmemittel (10) hinein erstreckt.

2. Vorrichtung nach Anspruch 1, wobei das erste Ende (4) ausgebildet ist, mit einem Kanülenansatz (11) in Eingriff zu treten.

3. Vorrichtung nach Anspruch 1 oder 2, wobei es sich bei dem Zellblockmaterial (2) um ein polymeres Material handelt.

4. Vorrichtung nach einem der Ansprüche 1 bis 3, wobei es sich bei dem Zellblockmaterial (2) um einen offenporigen Schaumstoff handelt.

5. Vorrichtung nach einem der Ansprüche 1 bis 4, wobei es sich bei dem Zellblockmaterial (2) um ein synthetisches Material handelt.

6. Vorrichtung nach Anspruch 5, wobei das Zellblockmaterial (2) ausgewählt ist aus Celluloseschwamm und PVA-Schaumstoff.

7. Vorrichtung nach Anspruch 6, wobei es sich bei dem Zellblockmaterial (2) um PVA-Schaumstoff handelt.

8. Vorrichtung nach einem der vorhergehenden Ansprüche, wobei das Zellblockmaterial (2) innerhalb des Gehäuses (3) so verschiebbar ist, dass es bei Verwendung in der Lage ist, weiter in das Gehäuse (3) hineinzugleiten, wenn das Probenentnahmemittel (10) mit der Vorrichtung (1) verbunden ist.

9. Vorrichtung nach einem der vorhergehenden Ansprüche, wobei das erste Ende (4) des Gehäuses (3) ausgebildet ist, mit dem Probenentnahmemittel (10) über eine Presspassung in Eingriff zu treten.

10. Vorrichtung nach einem der vorhergehenden Ansprüche, wobei das erste Ende (4) des Gehäuses (3) ausgebildet ist, mit dem Probenentnahmemittel (10) über einen Luer-Konus in Eingriff zu treten.

11. Vorrichtung nach einem der vorhergehenden Ansprüche, wobei das Gehäuse (3) ein zweites Ende (5) aufweist, das ausgebildet ist, über eine gasdichte Passung mit einer Spritzendüse (21) in Eingriff zu treten.

12. Vorrichtung nach Anspruch 11, wobei das zweite Ende (5) des Gehäuses (3) ausgebildet ist, mit der Spritzendüse (21) über eine Presspassung in Eingriff zu treten.

13. Vorrichtung nach Ansprüchen 11 oder 12, wobei das zweite Ende (5) des Gehäuses (3) ausgebildet ist, mit der Spritzendüse (21) über einen Luer-Konus in Eingriff zu treten.

14. Vorrichtung nach einem der vorhergehenden Ansprüche, wobei es sich bei dem Gehäuse (3) um ein röhrenförmiges Gehäuse handelt, welches das Zellblockmaterial (2) in Umfangsrichtung umgibt oder im Wesentlichen in Umfangsrichtung umgibt.

15. Vorrichtung nach Anspruch 2, wobei das Zellblockmaterial (2) im Wesentlichen eine zylinderartige Form aufweist und wobei sich bei Verwendung mit einer Kanüle (10) die Längsachse des Zylinders im Wesentlichen in der gleichen Richtung erstreckt wie die Längsachse eines an dem Kanülenansatz (11) befestigten Kanülenschafts (12).

## Revendications

1. Dispositif (1) comprenant un boîtier (3) et un matériau de bloc de cellules (2), dans lequel le boîtier (3) présente une première extrémité (4) qui est adaptée pour venir en prise avec un moyen d'échantillonnage (10) via un ajustement étanche aux gaz, et au moins une partie du matériau de bloc de cellules (2) est positionnée à l'intérieur du boîtier (3) de telle sorte que, lors d'une utilisation, le matériau de bloc de cellules (2) est en liaison fluidique avec le moyen d'échantillonnage (10),
**caractérisé en ce que** le matériau de bloc de cellules (2) fait saillie depuis la première extrémité (4) du boîtier (3) de telle sorte que, lors d'une utilisation, le matériau de bloc de cellules (2) s'étend dans le moyen d'échantillonnage (10).

2. Dispositif selon la revendication 1, dans lequel la première extrémité (4) est adaptée pour venir en prise avec un raccord d'aiguille (11).

3. Dispositif selon la revendication 1 ou 2, dans lequel le matériau de bloc de cellules (2) est un matériau polymère.

4. Dispositif selon l'une quelconque des revendications 1 à 3, dans lequel le matériau de bloc de cellules (2) est une mousse à cellules ouvertes.

5. Dispositif selon l'une quelconque des revendications 1 à 4, dans lequel le matériau de bloc de cellules (2) est un matériau synthétique.

6. Dispositif selon la revendication 5, dans lequel le matériau de bloc de cellules (2) est sélectionné parmi de l'éponge en cellulose et de la mousse PVA.

7. Dispositif selon la revendication 6, dans lequel le matériau de bloc de cellules (2) est de la mousse PVA.

8. Dispositif selon l'une quelconque des revendications précédentes, dans lequel le matériau de bloc de cellules (2) peut être coulissé à l'intérieur du boîtier (3) de telle sorte que, lors d'une utilisation, il est capable de coulisser plus loin dans le boîtier (3) lorsque le moyen d'échantillonnage (10) est raccordé au dispositif (1).

9. Dispositif selon l'une quelconque des revendications précédentes, dans lequel la première extrémité (4) du boîtier (3) est adaptée pour venir en prise avec le moyen d'échantillonnage (10) via un ajustement serré.

10. Dispositif selon l'une quelconque des revendications précédentes, dans lequel la première extrémité (4) du boîtier (3) est adaptée pour venir en prise avec le moyen d'échantillonnage (10) via un cône Luer.

11. Dispositif selon l'une quelconque des revendications précédentes, dans lequel le boîtier (3) présente une seconde extrémité (5) qui est adaptée pour venir en prise avec un embout de seringue (21) via un ajustement étanche aux gaz.

12. Dispositif selon la revendication 11, dans lequel la seconde extrémité (5) du boîtier (3) est adaptée pour venir en prise avec l'embout de seringue (21) via un ajustement serré.

13. Dispositif selon les revendications 11 ou 12, dans lequel la seconde extrémité (5) du boîtier (3) est adaptée pour venir en prise avec l'embout de seringue (21) via un cône Luer.

14. Dispositif selon l'une quelconque des revendications précédentes, dans lequel le boîtier (3) est un boîtier tubulaire qui entoure de manière circonférentielle ou entoure de manière sensiblement circonférentielle le matériau de bloc de cellules (2).

15. Dispositif selon la revendication 2, dans lequel le matériau de bloc de cellules (2) est formé sensiblement comme un cylindrique et dans lequel, lors d'une utilisation avec une aiguille (10), l'axe longitudinal du cylindre s'étend sensiblement dans la même direction que l'axe longitudinal d'une tige d'aiguille (12) fixée au raccord d'aiguille (11).
